# NOUVEAU FASCICULE DE BREVET EUROPEEN

(11) **EP 2 757 994 B2**
(45) Date de publication et mention de la décision concernant l'opposition: **24.08.2022**
(45) Mention de la délivrance du brevet: 21.01.2015
(21) Numéro de dépôt: 13808215.1
(22) Date de dépôt: 24.09.2013
(51) Int. Cl.: A61B 90/90, G06K 19/077, A61B 90/98

(54) **PROCÉDÉ D'ASSEMBLAGE D'UNE CAPSULE DE RADIO-IDENTIFICATION (RFID)**
VERFAHREN FÜR DIE MONTAGE EINER HOCHFREQUENZIDENTIFIKATIONSKAPSEL (RFID)
METHOD FOR THE ASSEMBLY OF A RADIO FREQUENCY IDENTIFICATION CAPSULE (RFID)

(30) Priorité: 24.09.2012 WO PCT/IB2012/055069
(43) Date de publication de la demande: 30.07.2014
(73) Titulaire: SATYATEK SA, 1800 Vevey (CH)
(72) Inventeur: BOVET, Marc, CH-2802 Develier (CH); ZIELASCH, Andreas, 82256 Fürstenfeldbruck (DE); GEHRIG, Nicolas, 1803 Chardonne (CH)
(74) Mandataire: Byrne, Declan
(86) Numéro de dépôt international: PCT/IB2013/058815
(87) Numéro de publication internationale: WO 2014/045265

(56) Documents cités:
- WO-A2-2009/063323
- DE-A1-102011 052 501
- DE-A1-102012 011 922
- DE-U1-202011 050 941
- US-A1- 2004 220 602
- US-A1- 2006 084 934
- US-A1- 2006 244 597
- US-A1- 2009 266 889
- US-A1- 2011 023 343
- US-A1- 2012 020 183
- US-A1- 2013 092 564

## Description

La présente invention concerne de manière générale une capsule de radio-identification (RFID) pour le marquage d'un objet et plus particulièrement le marquage RFID de dispositifs médicaux réutilisables et leur identification en vrac, automatique et multiple dans un environnement fortement métallique. L'invention s'applique aussi à d'autres objets, par exemple, des instruments dentaires, des implants, des substituts d'implants, des prothèses, des outils, et tout autre objets similaires que l'on souhaite marquer pour assurer son identification et sa traçabilité.

La traçabilité du processus de retraitement des dispositifs médicaux réutilisables permet de garantir qu'un instrument a été correctement retraité, c'est-à-dire: désinfecté, lavé, nettoyé, séché, contrôlé, emballé, et stérilisé avant une nouvelle utilisation sur un patient. Ce processus a pour but d'éviter la transmission d'agents infectieux aux patients.

La traçabilité à l'instrument permet d'informer les agent(e)s de stérilisation sur la manière de traiter les instruments (démontage, immersion, lavage, remontage, vérification de la fonctionnalité,...).

La traçabilité à l'instrument permet également d'améliorer la gestion financière et logistique des instruments (valorisation du stock, gestion des réparations et du remplacement des instruments, matério-vigilance, historique d'utilisation des instruments, stocks « dormants»...). Elle permet également le contrôle d'inventaire d'un panier de stérilisation et ainsi de réduire les risques d'incidents au bloc opératoire. En effet, un instrument manquant ou inadéquat peut remettre en cause le bon déroulement d'une opération chirurgicale et représente un risque pour la vie du patient. Une boîte incomplète ou incorrecte doit être renvoyée en stérilisation, ce qui implique un travail et donc des coûts supplémentaires.

Depuis 2010, la check liste OMS « sécurité du patient au bloc opératoire » impose le comptage des instruments avant et après l'opération. La présente invention permet l'identification en vrac, automatique et simultanée des instruments marqués et facilite la mise en place de cette directive tout en évitant les erreurs humaines et son impact sur les coûts en comparaison d'un comptage manuel par du personnel qualifié.

La traçabilité à l'instrument permet de maîtriser le risque de la Maladie de Creuzfeld - Jakob (MCJ) en identifiant les instruments utilisés chez des patients à risque et de bloquer, ainsi, des lots d'instruments infectés.

Le marquage RFID individuel des instruments permet de contrôler à la fin d'une opération qu'un instrument n'a pas été oublié dans le corps du patient ou jeté à la poubelle avec les drapages du bloc opératoire.

L'invention permet également d'empêcher la prise en charge d'instruments à ne pas stériliser (instruments à usage unique ou ayant atteint le nombre maximum de stérilisation).

Pour des raisons de coûts (temps d'opération) et sécuritaire (réduire, pour les agent(e)s de stérilisation, la manipulation d'instruments souillés) il est primordial que les instruments puissent être détectés, identifiés et comptés automatiquement et en vrac dans un panier de stérilisation en métal.

### Etat de la technique

Jusqu'à aujourd'hui, plusieurs technologies ont été utilisées pour résoudre en partie la problématique susmentionnée.

### Marquage couleur

Cette façon de faire ne permet pas d'attribuer un identifiant unique aux objets. Le système atteint rapidement ses limites car il faudrait marquer chaque objet avec une couleur différente, chose totalement impossible. Cette solution a néanmoins l'avantage de ne nécessiter aucun appareil particulier, Dans le cas où une pastille de même couleur est appliquée à l'ensemble des instruments d'un panier de stérilisation, le marquage couleur a l'avantage de permettre un tri rapide et manuel des instruments et d'aider à trier des instruments après leur utilisation au bloc opératoire.

Deux solutions existent :
- l'utilisation de bagues de couleur attachées à l'instrument, tel que décrit dans la demande de brevet WO8705487. Cette solution présente l'inconvénient de créer des nids à bactéries entre la bague de couleur et l'instrument, la liaison bague - instrument n'étant pas étanche ;
- soit la création d'une cavité remplie d'une résine colorée polymérisable, tel que décrit dans la demande de brevet WO2007090387. Cette solution a pour désavantage de modifier l'instrument et de créer des points de rupture potentiels.

### Identification optique, datamatrix, micro-percussion, gravure

Les codes optiques peuvent être appliqués sur les instruments soit par gravure au moyen d'un laser, soit par micro-percussion qui consiste en une succession d'impacts qui déforment la surface de la pièce à marquer.

Le marquage par code optique permet d'attribuer un numéro unique (codage normalisé de type GS1) ou un numéro interne choisi par son propriétaire et donc de reconnaître de manière unique deux objets se ressemblant en tous points.

L'identification de l'instrument ainsi marqué se fait au moyen d'un lecteur optique approprié. L'unicité d'un code n'est pas garantie car il est possible de graver un code un nombre de fois infini. Le brevet FR2899506 décrit une machine de codage datamatrix tandis que le brevet EP0681252A1 décrit une méthode pour marquer un instrument chirurgical.

Le marquage optique ne permet pas une identification automatique, multiple et en vrac des instruments marqués. Un code sale, mouillé ou dégradé ou tout élément opaque entre le code optique et le lecteur rendent la lecture impossible ou difficile. Un marquage profond de l'instrument le fragilise, un marquage superficiel disparaît facilement et doit être re-gravé souvent ce qui engendre des frais très importants.

### RFID

La technologie RFID permet :
i) d'attribuer un code unique à chaque dispositif médical et ainsi de pouvoir différencier de manière certaine et immédiate deux objets visuellement identiques ;
ii) d'identifier un instrument même s'il est emballé, sale ou mouillé;
iii) de modifier sans contact avec l'objet tout ou partie des données contenues dans la mémoire du tag RFID à l'exception de son code unique gravé lors de la fabrication. Ce code unique, lorsqu'il est présent, est infalsifiable. La mémoire du tag permet d'y stocker toute sorte d'information, par exemple un codage normalisé de type GS1.

Différents procédés de marquage par RFID ont été mis au point, les principales solutions techniques sont de :
- intégrer le tag RFID dans une cavité réalisée dans l'instrument tel que décrit dans les brevets WO2010145651 WO2009063323, WO2006067610, EP1774917.

Le premier inconvénient de cette solution réside dans le fait qu'il faille usiner une cavité dans l'instrument ce qui le fragilise et présente un risque de rupture de l'instrument lors d'un acte chirurgical. De plus, comme les instruments de chirurgie sont principalement en métal, le fait d'intégrer le tag RFID dans la matière réduit fortement la distance de lecture. L'identification multiple et en vrac n'est donc plus possible même dans un environnement non métallique.
- fabriquer une capsule, un bouton, une attache qui est ensuite fixé sur l'instrument à marquer.

Le document US20080177267 décrit une attache qui est fixée à l'instrument. Le plan de joint entre l'attache et l'instrument n'est pas étanche et offre des nids à bactéries potentiels. En outre, le nettoyage d'une telle pièce n'est pas aisé.

Le document US20060214791 décrit un tag bouton à visser sur l'instrument. Cette solution ne permet pas d'obtenir un plan de joint durablement étanche entre l'instrument et le bouton. Il est nécessaire de réaliser une cavité dans l'instrument pour pouvoir y visser le bouton.

Concernant la capsule décrite dans le document WO2011141912, le fait d'entourer le tag RFID par un anneau de métal en forme de « C » ne permet pas une identification en vrac, simultanée et automatique d'une centaine d'instruments dans un panier de chirurgie.

La technologie utilisée dans le document WO2011054355A2 ne permet pas une identification multiple, automatique et en vrac dans un panier chirurgical métallique. La capsule RFID est collée par application d'un mélange d'acrylates ou de méthacrylates polymérisables

Dans le document WO2013020944, deux tags sont nécessaires pour permettre une identification automatique, ce qui est un non-sens économique et démontre le manque d'efficacité de la technologie RFID choisie dans le cadre d'une utilisation en milieu fortement métallique. Par conséquent, une identification multiple, automatique et en vrac dans un panier en métal n'est pas possible.

Le document DE 20 2011 050941 U1 divulgue une capsule de radio-identification pour le marquage d'un objet, la capsule comprenant un socle métallique destiné à être fixé à l'objet et un logement.

### Inconvénients de l'état de la technique

Pour compter ou contrôler le contenu d'un panier de stérilisation, il faut identifier les instruments un à un ce qui prend beaucoup de temps. L'art antérieur ne fournit pas de solution permettant une identification multiple, automatique et en vrac d'instruments marqués dans un environnement fortement métallique, ni de solution de marquage versatile, hygiénique sans modification de l'instrument (exemples de paniers de stérilisation en métal: http://www.sterilmed.fr/avec-filligrane/sterilmed-02-paniers-et-accessoires-2012.pdf).

Un but de la présente invention est de répondre aux inconvénients mentionnés ci-dessus.

Plus précisément, le but de la présente invention est :
- premièrement, de proposer une capsule RFID pour instruments de chirurgie qui permet sa détection de manière automatique, en vrac et dans un environnement fortement métallique en présence de cages de Faraday. En effet jusque plus de 100 instruments peuvent être stockés dans des paniers de stérilisation en inox, leur chevauchement aléatoire et la présence, parfois, de bols en métal, créent forcément des cages de Faraday ;
- deuxièmement, de fournir une capsule RFID parfaite d'un point de vue médical et hygiénique aussi bien dans sa conception que dans son assemblage ;
- troisièmement, de fournir une capsule RFID permettant un assemblage rapide, permanent, résistant et étanche, sans nécessiter la réalisation d'une cavité dans l'instrument ;
- quatrièmement, de fournir une capsule RFID pouvant être assemblée sur une grande variété d'instruments ; et
- cinquièmement, de fournir une capsule RFID pouvant être fixée à des instruments neufs par le fabriquant et également à un parc d'instrument déjà existant, cela signifie que les instruments ne doivent pas être modifiés dans leur conception pour permettre leur marquage RFID.

La présente invention concerne ainsi un procédé d'assemblage d'une radio-étiquette dans une capsule de radio-identification selon la revendication 1.

La présente invention se compose d'un socle en métal, surmoulé d'un composite, le tout intégrant une radio-étiquette (tag RFID) et formant une capsule de radio-identification RFID. Cette capsule RFID peut être fixée de manière permanente et hygiénique à un dispositif médical pour lui donner une signature électronique unique, infalsifiable et consultable à distance.

D'autres caractéristiques et avantages de la présente invention apparaîtront plus clairement à la lecture de la description détaillée qui suit d'un mode de réalisation de l'invention donné à titre d'exemple nullement limitatif et illustré par les dessins annexés, dans lesquels :
- la Figure 1 illustre une vue en élévation d'une capsule de radio-identification ;
- la Figure 2 est une vue en coupe de la capsule de radio-identification ;
- la Figure 3a est une vue en coupe de la capsule de radio-identification selon l'axe A-A ;
- la Figure 3b est une vue en coupe similaire à celle de la Figure 3a dans laquelle le profil du fond du socle est illustré (voir la référence F de la Figure 1) ;
- la Figure 4 est une vue en coupe de la capsule de radio-identification comprenant un radioétiquette ;
- la Figure 5a illustre un socle de la capsule de radio identification ayant un chanfrein pour épouser un instrument 6 pans ;
- la Figure 5b illustre un socle de la capsule de radio-identification ayant un chanfrein pour épouser une partie d'un instrument d'un diamètre de 6mm ;
- la Figure 6 illustre une capsule de radio-identification assemblée sur une surface plate d'un instrument ;
- la Figure 7 illustre une capsule de radio-identification assemblée sur une surface non-plate d'un instrument ;
- la Figure 8 montre une vue en élévation et une vue en coupe d'une capsule de radio-identification selon une variante comportant un socle apte à épouser un instrument rond ;
- la Figure 9 montre une vue en élévation et une vue en coupe d'une capsule de radio-identification selon une variante comportant un socle apte à épouser un instrument 6 pans;
- la Figure 10 montre une vue en élévation et une vue en coupe d'une capsule de radio-identification selon une variante comportant un socle apte à épouser un instrument sur une surface plate;
- la Figure 11 est une vue en élévation d'une capsule de radio-identification selon une variante comportant un socle apte à épouser un instrument rond ; et
- la Figure 12 est une vue en élévation d'une capsule de radio-identification selon une autre variante comportant un socle apte à épouser un instrument 8 pans.

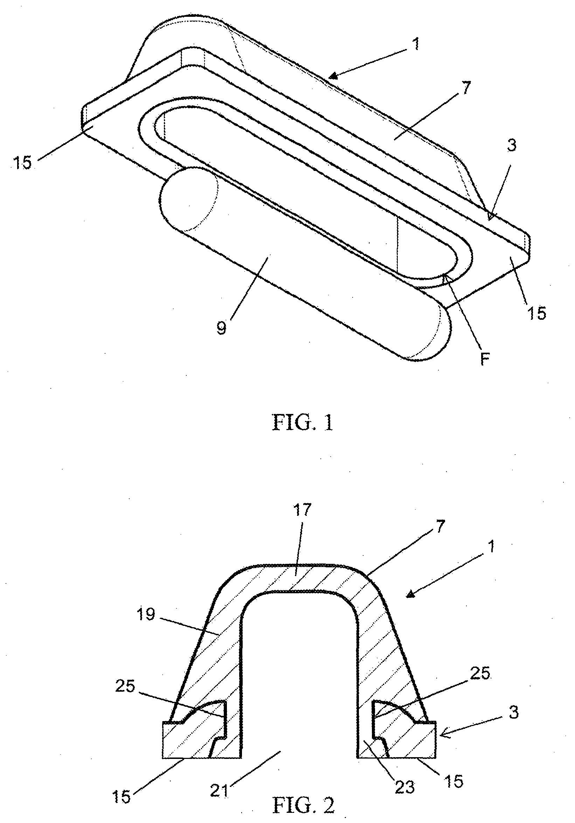

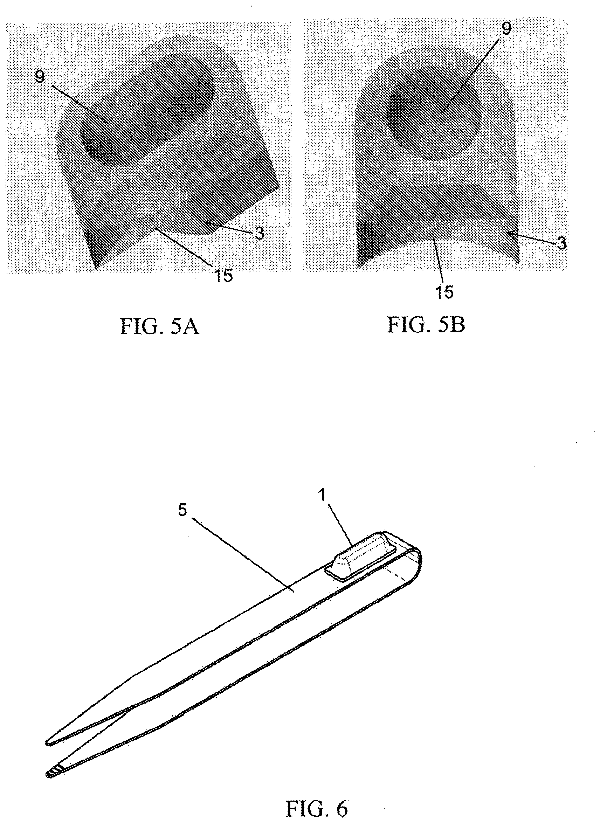

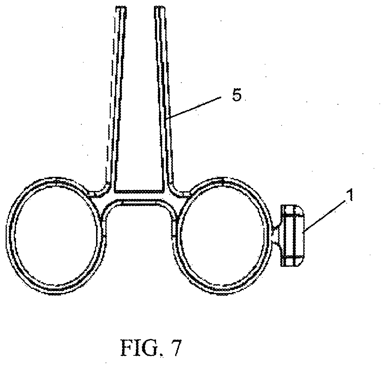

Les Figures 1 et 2 illustrent une capsule de radio-identification 1 (capsule RFID) comprenant un socle (ou un support) métallique 3 destiné à être fixé à un objet 5 à marquer (Figures 6 et 7) et un logement 7 apte à détenir un radio-étiquette (tag RFID) 9. Le logement 7 est solidaire du socle et est surmoulé sur le socle 3 pour former un joint durablement étanche avec le socle 3.

Le socle est, par exemple, réalisé en inox (inox 316L). Le logement 7 est réalisé en polyétheréthercétone (PEEK) renforcé en fibre de verre ou fibre de carbone.
La réalisation en polyétheréthercétone (PEEK) peut être faite en couleurs différentes.

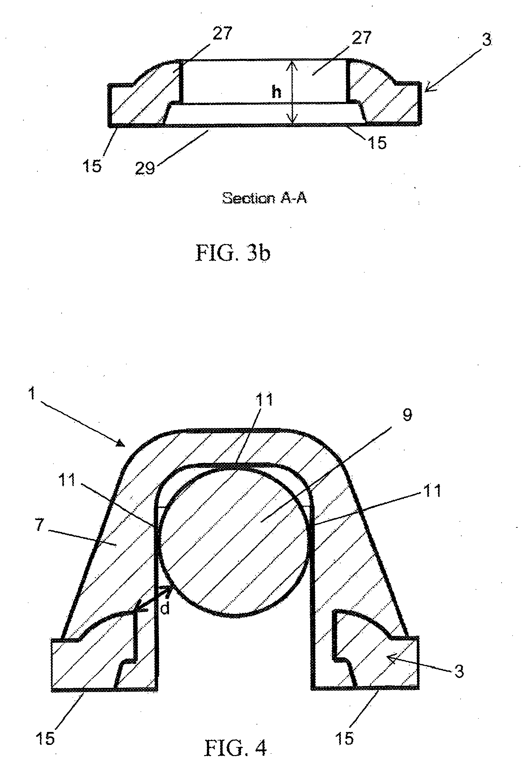

La capsule de radio-identification 1 comprend en outre des moyens 11 pour tenir la radio-étiquette 9 à une distance fixe **d** du socle 3 (Figure 4), la distance d étant la distance la plus courte entre le socle 3 et la radioétiquette 9.

Les moyens 11 pour tenir la radio-étiquette 9 à une distance fixe du socle peuvent être des clips (pinces) présents dans le logement 7 attachés à une surface intérieure du logement 7 et apte à recevoir la radio-étiquette 9, un collage de la radio-étiquette 9 à la surface intérieure du logement 7, un remplissage du logement par un matériau de remplissage (par exemple, une résine ou autre matériau de remplissage) ou un clipsage (par exemple, par enfoncement, collage ou soudage) d'une contre-pièce de préhension (en en composite en métal et dans la cavité du logement) dont la forme reproduit en négatif le profil de la radio-étiquette 9.

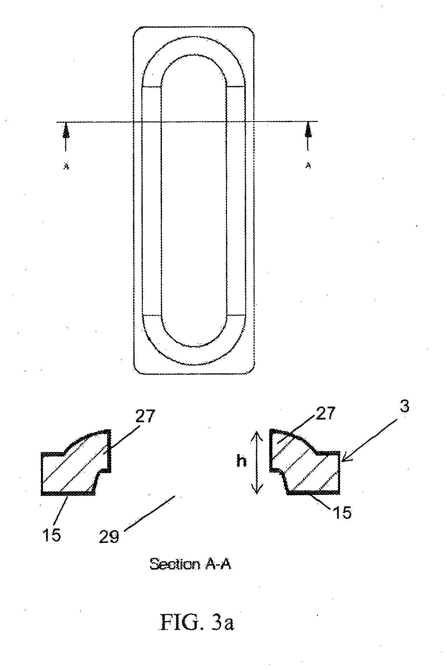

Le logement 7 comprend un fond 17, une paroi latérale 19 reliée au fond 17 et délimitant une ouverture 21 pour recevoir la radio-étiquette 9 dans le logement 9. Le logement 7 inclut en outre un bord 23 à l'extrémité de la paroi 19 incluant un évidement 25. Le socle 3 comporte un décrochement 27 (Figure 3) configuré pour coopérer avec l'évidement 25 permettant de solidariser le logement 7 au socle 3. L'évidement 25 permet une accroche mécanique optimale au socle du matériau d'encapsulation du logement 7.

Le socle 3 a une hauteur h par rapport à sa surface extérieure 15 d'au moins 0,5mm (Figure 3) afin que le matériau d'encapsulation du logement 7 ne soit pas endommagé lors du processus de soudage par laser. Le socle inclut en outre une ouverture 29 permettant l'insertion de la radio-étiquette 9 dans le logement 7 après le surmoulage et la fabrication de la capsule.

La surface extérieure 15 peut prendre différentes formes en fonction de la géométrie de l'objet à marquer. La surface extérieure 15 du socle 3 destiné à être fixé sur un instrument 5 peut être plate, courbée, dotée d'un angle sensiblement au milieu de la surface du socle (Figure 5A), dotée d'un polyèdre ou a une forme sensiblement circulaire d'un rayon prédéterminé (Figure 5B) pour bien épouser la forme de la partie de l'instrument 5 à laquelle la capsule sera attachée.

Dans les modes de réalisation illustrés sur les Figures 5A, 5B et 8 à 9 et par rapport au socle 3 du mode de réalisation illustré dans les Figures 1 à 4, le socle 3 comporte en outre une base 31 fermant le logement 7, la base 31 comportant une surface extérieure 35 destinée à être fixée sur un instrument 5. La base 31 est soudée sur la surface 15 par l'intermédiaire d'un laser ou collée à la surface 15.

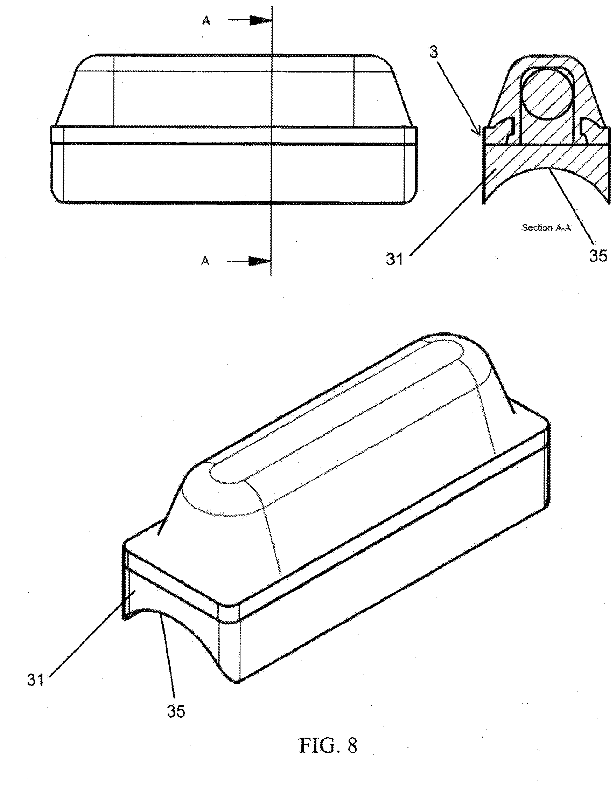

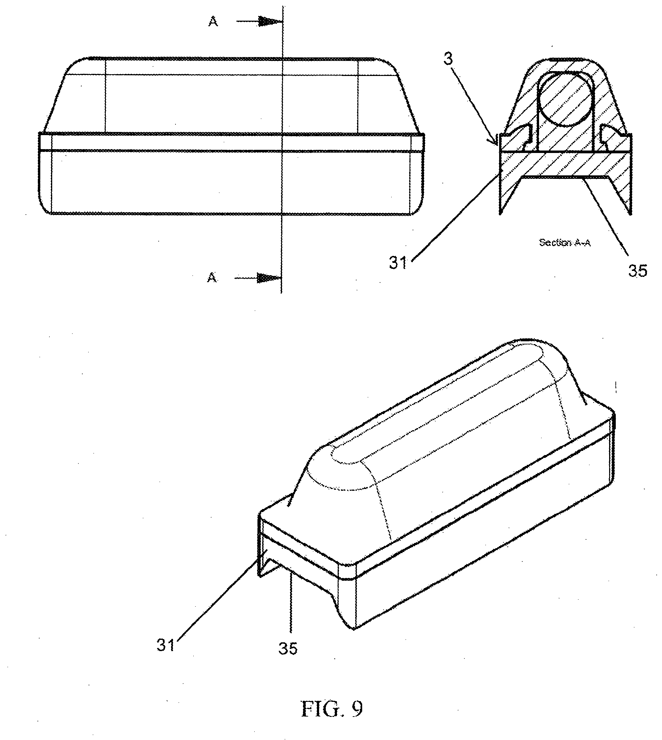

Cette surface extérieure 35 peut être plate, courbée, dotée d'un angle sensiblement au milieu de la surface extérieure 35, dotée d'un polyèdre ou a une forme sensiblement circulaire d'un rayon prédéterminé (Figure 8) pour bien épouser la forme de la partie de l'instrument 5 à laquelle la capsule sera attachée. La Figure 9 montre une capsule 1 comportant un socle ayant une surface extérieure 35 apte à épouser un instrument 6 pans.

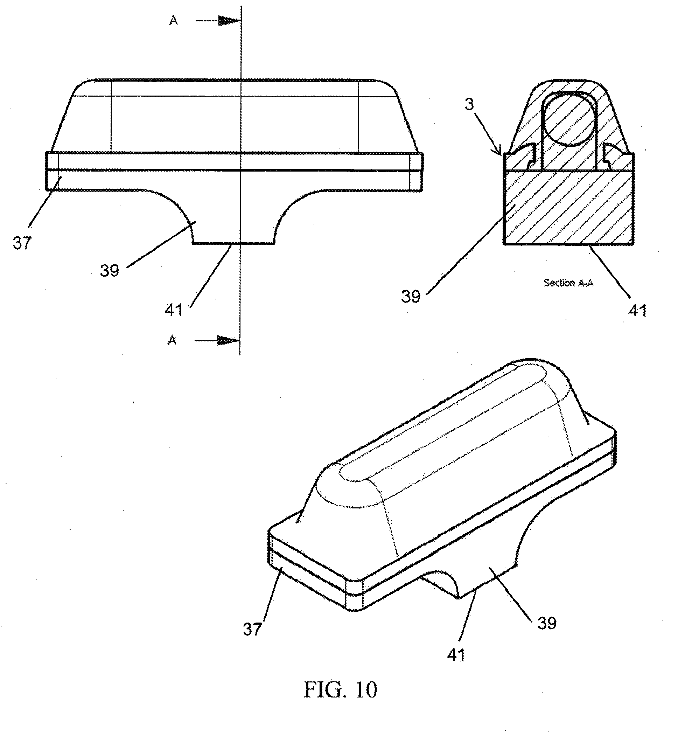

Dans les modes de réalisation illustrés sur les Figures 10 à 12 et par rapport au socle 3 du mode de réalisation illustré dans les Figures 1 à 4, le socle 3 comporte en outre une base 37 fermant le logement 7 ainsi qu'un ergot 39 ayant une surface extérieure 41 destinée à être fixée sur un instrument 5. La base 37 est soudée sur la surface 15 par l'intermédiaire d'un laser ou collée à la surface 15.

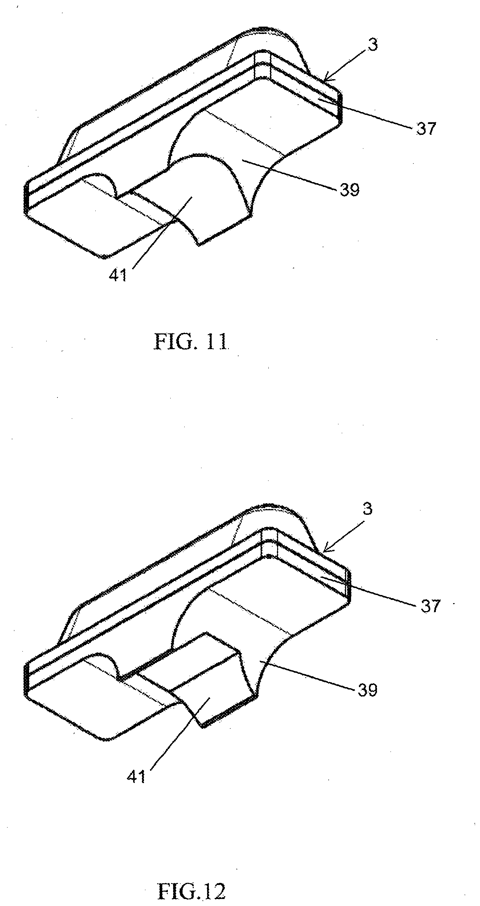

Cette surface extérieure 41 peut être plate (Figure 10), courbée, dotée d'un angle sensiblement au milieu de la surface extérieure 41, dotée d'un polyèdre ou a une forme sensiblement circulaire d'un rayon prédéterminé (Figure 11) pour bien épouser la forme de la partie de l'instrument 5 à laquelle la capsule sera attachée. La Figure 12 montre une capsule 1 comportant un socle ayant une surface extérieure 41 apte à épouser un instrument 8 pans.

Le matériau d'encapsulation préféré pour surmouler le logement 7 sur le socle 3 en métal est un PEEK chargé en fibres de verre ou fibres de carbone pour les raisons suivantes :
- Résistance mécanique, notamment
   i. Chocs courants pour éviter le bris du tag RFID 9
   ii. Résistance aux coupures
   iii. Résistance aux frottements
   iv. Aux bains à ultrasons
- Résistance chimique, notamment
   i. Soude caustique à concentration molaire 1 à 2 exposition
   ii. Acide ortho-phosphorique (par exemple : Deconex 34 Borer)
   iii. Détergents dans les laveurs-désinfecteurs (par exemple : Deconex 23 Borer)
- Résistance thermique
   i. Au moins 1500 cycles en autoclave Prion (134°C, 18 min, 3 bars de pression sous atmosphère saturée de vapeur d'eau déminéralisée).
   ii. Exposition à une température de 200°
- Compatibilité biomédicale
   i. Matériau cyto-compatible, éventuellement biocompatible
   ii. Surface lisse sans nids à bactéries
   iii. Plan de joint étanche entre le matériau de surmoulage et le socle en métal 3
   iv. Durabilité de l'étanchéité garantie grâce aux coefficients de dilation thermiques proches entre le socle en métal 3 et le matériau d'encapsulation du logement 7
- Possibilité de colorer la matière d'encapsulation dans différentes couleurs
- Le matériau d'encapsufation ne se termine pas au bord du socle en métal 3 pour permettre la fermeture du moule d'injection lors du surmoulage.
   Cette propriété permet également d'éviter une détérioration du matériau d'encapsulation lors du soudage laser de la capsule RFID 1 sur un instrument 5.

La radio-étiquette (tag RFID) 9 est insérée dans la capsule 1 après le surmoulage.

Dans le cadre de l'utilisation d'une radio-étiquette 9 (tags RFID) basse fréquence LF ou ultra hautes fréquences UHF, la distance fixe entre la radio-étiquette 9 et le socle 3 en métal ainsi que la fréquence de la radio-étiquette 9 permettent de créer un environnement normalisé au niveau du champ magnétique. La position de la radio-étiquette 9 par rapport au socle 3 est variée et une fréquence de fonctionnement de la radio-étiquette 9 est déterminée afin d'obtenir une position et une fréquence auxquelles la fréquence de fonctionnement de la capsule 1 soit entre 121kHz et 129kHz (et de préférence à 125kHz). Il est ainsi possible de spécifier et de garantir des performances RFID optimales (distance et fiabilité de lecture) en fonction de la matière de l'objet sur lequel la capsule RFID 1 sera fixée.

Si une radio-étiquette 9 LF est utilisée, il est à relever que la distance d entre la radio-étiquette 9 et le socle 3 en métal doit être fixée par les moyens 11 à une valeur d'environ 0,2mm, par exemple. La fréquence de résonnance de la radio-étiquette 9 est adaptée en fonction du métal utilisé pour qu'une fois en présence du socle 3 métallique, la capsule 1 fonctionne à une valeur normalisée entre 121 kHz et 129kHz (et de préférence d'environ 125khz (ou à 125kHz)) avant l'assemblage de la capsule 1 sur l'objet 5 et lorsque la capsule est assemblée sur l'objet 5 à marquer.

En effet, si l'on applique une radio-étiquette 9 directement sur une surface métallique, les effets divers de detuning rendraient une détection de la radio-étiquette 9 soit impossible ou la distance de détection serait trop réduite pour permettre une identification en vrac et automatique dans un panier de stérilisation métallique: La stratégie de « normalisation » ci-dessus permet d'automatiser la détection d'objets marqués par la capsule RFID 1 et de conserver une détection instantanée et automatique d'objets placés en vrac dans un panier de stérilisation lequel est placé dans un tunnel RFID tel que décrit dans la demande WO 2010/109412 A1 intitulée SYSTEME ET PROCEDE POUR LA LECTURE D'UN OU DE PLUSIEURS TAGS RFID EN MODE ANTICOLLISION DANS UNE CASSETTE METALLIQUE ET EN DEHORS.

De préférence, des radio-étiquettes 9 passives fonctionnant à basse fréquence 35 - 150 kHz sont utilisées.

De préférence, la radio-étiquette 9 se présente sous la forme d'un objet cylindrique comprenant :
- Un transpondeur ou chip RFID
- Une ferrite (de forme cylindrique, en E, en U ou incurvée)
- Une bobine de cuivre soudée au transpondeur
et éventuellement un tube en verre protégeant les éléments ci-dessus.

Ces radio-étiquette 9 se dénomment usuellement « glasstags » ou « metal rod tag » et existent dans différentes dimensions et exécutions. Ils peuvent également être équipés de différents types de transpondeurs notamment en basse (LF) ou haute fréquence (HF). Ils sont notamment commercialisés par la société HID Global sous cette dénomination.

La présente invention permet également de s'adapter à des radio-étiquettes UHF de petites dimensions, tels que celles proposées par la société Xerafy. Ils sont notamment commercialisés sous la dénomination Dot-On XS et Dash-On XS.

Des radio-étiquettes équipées de bobine aérienne (air coil) peuvent également être utilisées, ces radio-étiquettes se présentent généralement sous forme de disques. Elles sont notamment commercialisées par la société HID Global sous le nom commercial Micro-Prox^{™}tag, (voir le lien : http://www.hidglobal.com/main/id-cards/hid-proximity/1391-microproxtag.html).

Pour d'autres objets à équiper de taille plus grande que les instruments ou outils, par exemple des containers, chariot ou palettes des radio-étiquettes RFID d'autres formes et fonctionnant avec d'autres fréquences (HF, UHF, MW) peuvent être utilisées. Elles sont notamment commercialisées par la société HID Global sous le nom commercial InLine UHF^{™} tag.

Les radio-étiquettes RFID utilisées (glasstags LF) résistent à plus de 1500 cycles de stérilisation Prion et elles résistent aux bains ultrasons.

La puce utilisée dans la radio-étiquette 9 possède un algorithme anti-collision pour identifier un à un les objets d'une collection d'objets lors de lectures multiples.

Le fait que tout ou partie de l'objet à marquer soit en métal n'empêche pas d'identifier un objet parmi la collection d'objets.

La présence de métal n'empêche pas la détection automatique d'objets parmi une collection d'objets.

Les objets marqués au moyen de la capsule de la présente invention peuvent être déposés dans un panier de stérilisation en inox, un plateau chirurgical, dans une cassette métallique fermée, un « haricot », un panier de stérilisation et il est possible de toujours détecter les objets parmi la collection d'objets.

On comprendra que diverses modifications et / ou améliorations évidentes pour l'homme du métier peuvent être apportées aux différents modes de réalisation de l'invention décrits dans la présente description sans sortir du cadre de l'invention défini par les revendications annexées.

## Revendications

1. Procédé d'assemblage d'une radio-étiquette (9) dans une capsule (1) de radio-identification pour le marquage d'un dispositif médical, ou d'un dispositif médical réutilisable, ou d'un instrument de chirurgie ou d'un instrument dentaire (5) pour fournir une capsule (1) de radio-identification fonctionnant entre 121kHz et 129kHz lorsque la capsule est assemblée sur un objet (5) et pour former un environnement normalisé permettant la détection de l'objet (5) dans un panier de stérilisation en métal comprenant une pluralité d'objets (5), la capsule (1) comprenant :
- un socle (3) métallique destiné à être fixé à l'objet (5) ;
- un logement (7) apte à détenir une radio-étiquette (9), le logement (7) étant solidaire au socle (3) et surmoulé sur le socle (3) afin de former un joint durablement étanche avec le socle (3) ;
- des moyens (11) pour tenir la radio-étiquette à une distance fixe du socle (3),
le logement (7) étant réalisé en polyétheréthercétone (PEEK) renforcé en fibre de verre ou de carbone ;
le procédé comprenant les étapes de :
- insérer une radio-étiquette (9) dans le logement (7) de la capsule (1) ;
- varier la position de la radio-étiquette (9) par rapport au socle (3) et définir sa fréquence de fonctionnement pour déterminer une position et une fréquence auxquelles la fréquence de fonctionnement de la capsule (1) soit entre 121kHz et 129kHz ; et
- fixer la radio-étiquette (9) à cette position déterminée en utilisant les moyens (11) pour tenir la radio-étiquette à une distance fixe du socle.

## Patentansprüche

1. Verfahren zur Montage eines Hochfrequenzetiketts (9) in einer Hochfrequenzidentifikationskapsel (1) zum Markieren einer medizinischen Vorrichtung oder einer wiederverwendbaren medizinischen Vorrichtung oder eines chirurgischen Instruments oder eines zahnärztlichen Instruments (5), um eine Hochfrequenzidentifikationskapsel (1) zu liefern, die zwischen 121 kHz und 129 kHz funktioniert, wenn die Kapsel auf einem Objekt (5) montiert wird, und um eine genormte Umgebung zu bilden, die die Erfassung des Objekts (5) in einem Sterilisationskorb aus Metall, umfassend eine Vielzahl von Objekten (5), ermöglicht, wobei die Kapsel (1) umfasst:
- einen Metallsockel (3), der dazu bestimmt ist, am Objekt (5) befestigt zu werden;
- eine Lagerung (7), die geeignet ist, ein Hochfrequenzetikett (9) zu enthalten, wobei die Lagerung (7) mit dem Sockel (3) verbunden und auf dem Sockel (3) aufgeformt ist, um eine dauerhaft dichte Verbindung mit dem Sockel (3) zu schaffen;
- Mittel (11), um das Hochfrequenzetikett in einer festen Distanz zum Sockel (3) zu halten,
wobei die Lagerung (7) aus Polyetheretherketon (PEEK), das mit Glas- oder Kohlenstofffaser verstärkt ist, hergestellt ist,
wobei das Verfahren die folgenden Schritte umfasst:
- Einsetzen eines Hochfrequenzetiketts (9) in die Lagerung (7) der Kapsel (1);
- Variieren der Position des Hochfrequenzetiketts (9) in Bezug zum Sockel (3) und Definition seiner Funktionsfrequenz, um eine Position und eine Frequenz zu bestimmen, bei denen die Funktionsfrequenz der Kapsel (1) zwischen 121 kHz und 129 kHz liegt; und
- Befestigen des Hochfrequenzetiketts (9) in dieser bestimmten Position unter Verwendung der Mittel (11) zum Halten des Hochfrequenzetiketts in einer festen Distanz zum Sockel.

## Claims

1. Method of fitting a radio-tag (9) in a radio-identification capsule (1) for the marking of a medical device, or of a reusable medical device, or of a surgical instrument or a dental instrument so as to provide a radio-identification capsule (1) operating between 121kHz and 129kHz when the capsule is fitted on an object (5) and to form a standardized environment allowing the detection of the object (5) in a metal sterilization basket comprising a plurality of objects (5), the capsule (1) comprising:
- a metallic mount (3) intended to be fixed to the object (5);
- a housing (7) able to retain a radio-tag (9), the housing (7) being secured to the mount (3) and overmoulded on the mount (3) so as to form an enduringly leaktight seal with the mount (3);
- means (11) for holding the radio-tag at a fixed distance from the mount (3),
the housing (7) is made of polyetheretherketone (PEEK) reinforced with carbon or glass fibre;
the method comprising the steps of:
- inserting a radio-tag (9) into the housing (7) of the capsule (1);
- varying the position of the radio-tag (9) with respect to the mount (3) and defining its operating frequency so as to determine a position and frequency at which the operating frequency of the capsule (1) is between 121kHz and 129kHz; and
- fixing the radio-tag (9) at this determined position by using the means (11) for holding the radio-tag at a fixed distance from the mount.
